# EUROPEAN PATENT APPLICATION

(11) **EP 4 751 671 A1**
(43) Date of publication of application: **03.06.2026**
(21) Application number: 25215782.1
(22) Date of filing: 14.11.2025
(51) Int. Cl.: A61B 18/14, A61B 5/00, A61B 5/06

(54) **CATHETER CONTACT FORCE ESTIMATION DURING ABLATION**

(30) Priority: 02.12.2024 US 202418965306
(71) Applicant: Biosense Webster (Israel) Ltd., 2066717 Yokneam (IL)
(72) Inventor: GLINER, Vadim, 2066717 Yokneam (IL); GOVARI, Assaf, 2066717 Yokneam (IL)
(74) Representative: Carpmaels & Ransford LLP

(57) **Abstract**

In one example, an apparatus includes an ablation energy generator to conduct ablative energy to an electrode of a catheter, a processor configured to compute a first force signal acting on tissue by a distal end assembly of the catheter based on a first magnetic signal received by a first magnetic field sensor of the distal end assembly from a magnetic transmitter coil of a distal end of the catheter during ablation of tissue by the electrode, compute a second force signal acting on tissue by the distal end assembly based on a second magnetic signal received by a second magnetic field sensor of the distal end assembly from a magnetic transmitter coil disposed in a location pad, compute a third force signal based on the first and second force signal, and render to a display a force value or an ablation index value computed from the third force signal.

## Description

### FIELD OF THE DISCLOSURE

The present disclosure relates to medical systems including catheters, and in particular, but not exclusively to, catheter contact force estimation.

### BACKGROUND

A wide range of medical procedures involve inserting probes, such as catheters, within a patient's body. One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Catheters are inserted into the heart chamber and optionally around the heart chamber during such procedures. In most procedures, multiple catheters are inserted into the patient. Catheters may include mapping, ablation, temperature sensing and image sensing catheters. Some catheters are dedicated for placement in specific parts of the anatomy, e.g., coronary sinus, esophagus, atrium, ventricle. The catheters have multiple electrical channels, some more than others depending on the number of sensors and electrodes included in each catheter. The number and type of catheters depends on the procedure and on the physician preferred workflow.

### BRIEF DESCRIPTION OF THE DRAWINGS

The present disclosure will be understood from the following detailed description, taken in conjunction with the drawings in which:
Fig. 1 is a simplified illustration of a catheter-based electrophysiology mapping and ablation system constructed and operative in accordance with an example of the present disclosure;
Fig. 2 is a schematic view illustrating sensing magnetic signals from a shaft-based magnetic coil for use in the system of Fig. 1;
Fig. 3 is a schematic view illustrating sensing magnetic signals from a location pad magnetic coil for use in the system of Fig. 1;
Fig. 4 is a flowchart including steps in a method of operation of the system of Fig. 1;
Fig. 5 is a schematic view of a Kalman filter combining a first and second force signal into a third force signal; and

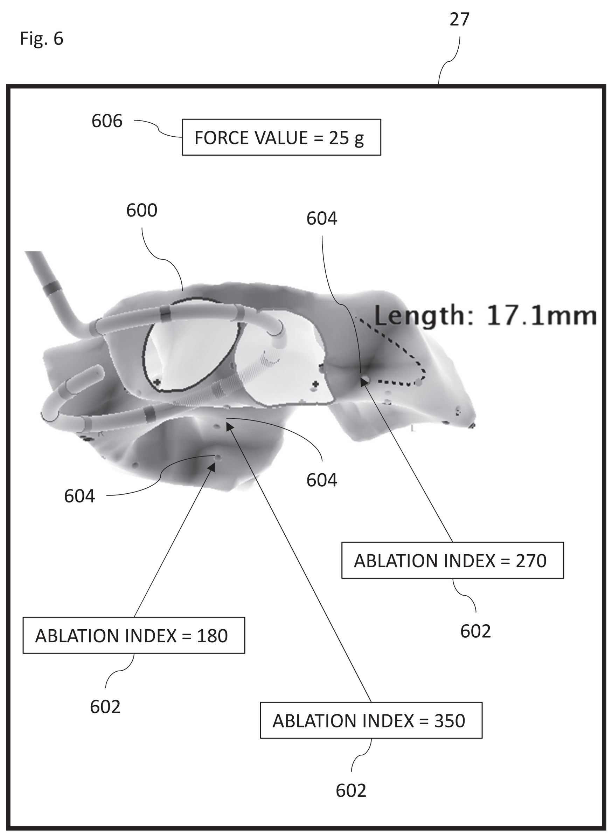

Fig. 6 is a schematic view of a display showing an electro-anatomical map and various related values.

### DESCRIPTION OF EXAMPLES

### OVERVIEW

During electro-anatomical mapping and ablation, it is important that the physician positions the catheter so that the relevant electrodes have sufficient contact with the cardiac tissue, and press against the tissue with a sufficient force. This is particularly important during ablation, as in order to provide a successful ablation, the force that the electrode exerts on the tissue should be above a given limit.

The force may be measured using one or more force sensors built into the catheter. Force sensors may provide an acceptable solution when the catheter is a focal catheter, but when the catheter includes multiple electrodes on a distal end assembly (e.g., a basket or balloon shape) which is deformable, the force sensors may not provide an optimal solution and/or be difficult to implement.

One method to determine the forces exerted by the electrodes may be estimated based on sensing a magnetic field generated by a magnetic coil in the distal end of the shaft of the catheter. The magnetic field is sensed by magnetic sensors disposed on the distal end assembly. During calibration different measured forces may be applied to the catheter and the magnetic field sensed at each magnetic sensor is measured thereby creating a mapping between the sensed magnetic field and the forces. The mapping may then be used to find the force exerted by the electrodes based on the sensed magnetic field by the magnetic sensors.

However, during ablation, especially with short pulses of very high ablation energy (e.g., pulsed field ablation (PFA)), the ablation signals create a lot of noise at the magnetic transmission coil(s) as the wires for the ablation run through, or close to, the magnetic transmission coil(s) and interfere with magnetic transmission coil(s) and cause the magnetic transmission coil(s) to generate frequencies that the magnetic transmission coil(s) are not designed to generate.

Therefore, using the magnetic transmission coil(s) to provide the magnetic signals during ablation may lead to inaccuracies when computing the positions of the sensors (or of other elements) and/or the shape of the distal end assembly, and the force(s) exerted on the tissue by the electrode(s) of the distal end assembly.

As the distal end assembly has elastic properties another method to determine the forces exerted by the electrodes may be estimated based on the deformed shape of the distal end assembly using a predefined linear or non-linear elastic model, as the reason the distal end assembly becomes deformed is due to the forces applied by the surrounding tissue on the distal end assembly. The different deformed shapes of the distal end assembly may be calibrated to measured forces. The distal end assembly may be deformed in multiple ways and the resulting forces are measured, thereby providing a mapping between the different deformed shapes of the distal end assembly and the resulting forces. In another example, the forces of the different deformed shapes of the distal end assembly may be computed based on a model of the distal end assembly and the structural properties of the materials used to form the distal end assembly.

During in-vivo use of the catheter, the deformation of the distal end assembly may be tracked based on the positions of one or more magnetic field sensors (e.g., on the distal end assembly (such as on splines of a basket catheter) and in the distal end of the shaft of the catheter). The associated force(s) may be determined as a deformation of the distal end assembly, using any suitable method, such as the mapping described above.

The positions of the magnetic field sensors may be computed based on magnetic signals transmitted from magnetic transmitter coils disposed in a location pad positioned ex-vivo, around the patient's body (e.g., around the chest). Due to the larger distance from the location pad to the distal end of the catheter, the location pad method does not suffer from the noise caused by the ablation. However, due to the relatively large distance of the location pad from the magnetic field sensor(s), the forces computed from the deformation of the distal end assembly are generally determined with a lower degree of accuracy, than with the abovementioned method based on sensing magnetic field(s) from a transmitter in the shaft of the catheter.

Therefore, exemplary modes of the present disclosure address the above drawbacks by computing a force signal based on the above two methods, particularly when ablation is being performed. The magnetic signal(s) sensed from the magnetic transmitter coil(s) disposed in the distal end of the catheter are used to compute a time varying force signal (referred to as a first force signal). The magnetic signal(s) from the magnetic transmitter coils disposed in the location pad are used to compute the positions of the magnetic field sensor(s) (or other elements), the deformation of the distal end assembly over time from the computed positions of the magnetic field sensor(s), and from the deformation a time varying force signal (referred to as a second force signal) is computed. A third force signal is computed from the first force signal and second force signal, for example, by combining information from the first force signal and the second force signal, to better estimate the force exerted by one or more of the electrodes on the cardiac tissue.

When ablation energy is not being delivered, e.g., during idle mode, the system typically uses the first force signal, without the second force signal, for example to compute ablation indices or forces to be displayed for the physician to view.

In some examples, a Kalman filter receives the first and second force signals as input and combines information from the first and second force signal to yield the third force signal. The Kalman filter provides a mathematical optimal estimation of the force signal by selecting between the accurate but not reliable values and the reliable but not accurate values.

In some examples, a neural network may be trained to find the third force signal from the first and second forces signals based on using force values when no ablation is being performed to train the network.

In some examples, force values from the third force signal may be displayed for the physician to view during ablation. The third force signal may also be used with other suitable values (e.g., ablation power and optionally number of ablation pulses) to compute one or more ablation index values based on real-time force sensing during ablation, for example, at different respective ablation sites on the cardiac tissue. In some examples, the computed ablation index values may be recorded and associated with respective ablation sites in an electro-anatomical map for selective display.

### SYSTEM DESCRIPTION

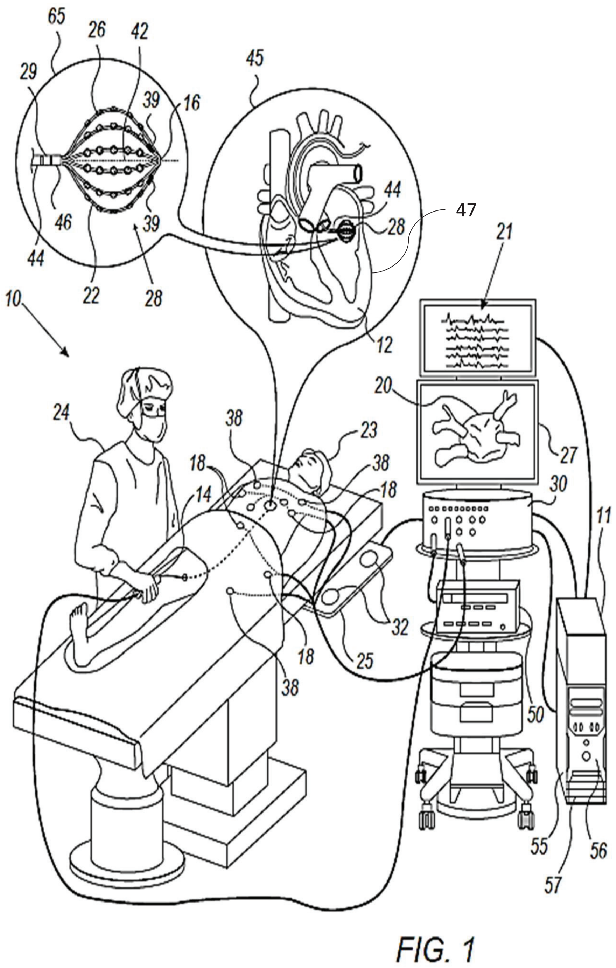

Reference is now made to Fig. 1, which is a schematic, pictorial illustration of a catheter-based electroanatomical (EA) mapping and ablation system 10, in accordance with an example of the present disclosure. System 10 includes one or more catheters, which are percutaneously inserted by physician 24 through the patient's vascular system into a chamber or vascular structure of a heart 12. Typically, a delivery sheath catheter is inserted into the left or right atrium near a desired location in heart 12. Thereafter, one or more catheters in turn can be inserted into the delivery sheath catheter to arrive at the desired location. The one or more catheters may include catheters dedicated for sensing intracardiac electrogram (IEGM) signals, catheters dedicated for ablating and/or catheters dedicated for both sensing and ablating. An example basket catheter 14 that is configured for sensing IEGM is illustrated herein. As seen in inset 45, physician 24 brings a basket type of expandable distal end assembly 28 (also called hereinafter "expandable distal-end assembly 28") fitted on a shaft 44 of catheter 14 into contact with the heart wall for sensing a target site in heart 12. For ablation, physician 24 similarly brings the distal end of an ablation catheter to a target site for ablating.

As seen in inset 65, catheter 14 is an exemplary catheter that includes one, and preferably multiple, electrodes 26 optionally distributed over a plurality of splines 22 at expandable distal-end assembly 28 and configured to sense IEGM signals. Catheter 14 additionally includes (i) a proximal magnetic field sensor 29 (e.g., a dual axis sensor (DAS) comprising two electro-magnetic coils (EMCs) or a triple axis sensor (TAS) 29 comprising three EMCs) embedded in a distal end 46 of shaft 44 near expandable distal end assembly 28, and (ii) two distal magnetic field sensors 39 (e.g., single axis sensor (SAS) 39 comprising a single EMC) to track the position of the distal end of expandable distal end assembly 28. Optionally, and preferably, magnetic field sensors 29 and 39 are magnetic field sensors that include magnetic coils for sensing three-dimensional (3D) position.

System 10 includes one or more electrode patches 38 positioned for skin contact on patient 23 to establish a location reference for location pad 25 as well as impedance-based tracking of electrodes 26.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablating. Energy produced by ablation energy generator 50 may include, but is not limited to, radio frequency (RF) energy or pulsed-field ablation (PFA) energy, including monopolar or bipolar high-voltage DC pulses that may be used to effect irreversible electroporation (IRE), or combinations thereof.

Patient interface unit (PIU) 30 is a controller with processing capability that is configured to establish electrical communication between catheters, electrophysiological equipment, power supply and a workstation 55 for controlling the operation of system 10. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, processor unit 56 with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

### TWO ALTERNATIVE METHODS FOR COMPUTING FORCE OF CATHETER EXPANDABLE ASSEMBLY

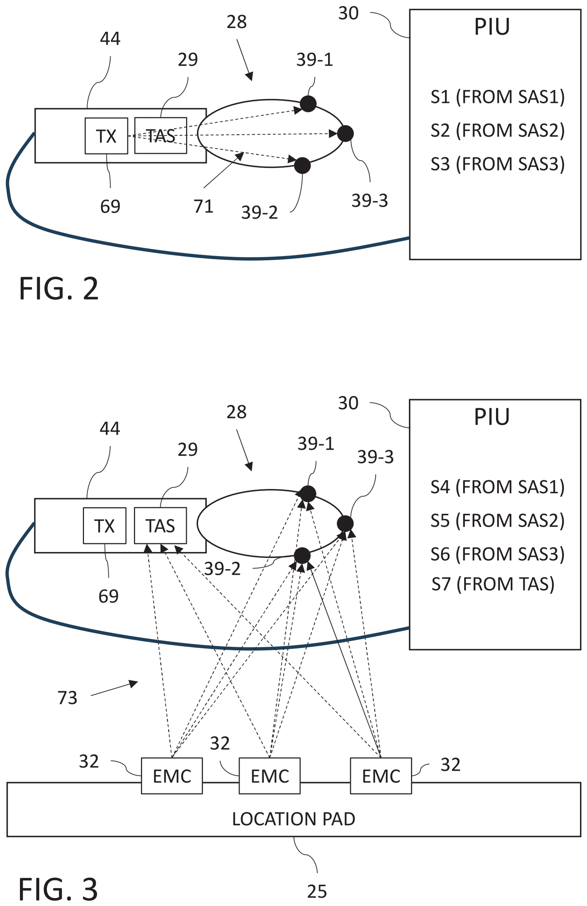

Reference is now made to Fig. 2, which is a schematic view illustrating sensing magnetic signals 71 from a magnetic transmitter coil 69 for use in the system 10 of Fig. 1. In some examples, a shaft-based magnetic coil 29 (e.g., a TAS) may transmit the magnetic signals 71. A more accurate method to estimate the force exerted by the distal end expandable assembly 28 includes magnetic transmitter coil 69 transmitting magnetic signals 71 for detection by magnetic field sensors 39. Fig. 2 shows three magnetic field sensors 39, for example, single axis sensors (SAS) such as SAS1 39-1, SAS2 39-2, and SAS3 39-3. SAS1 39-1 receives signal S1, SAS2 39-2 receives signal S2, and SAS3 39-3 receives signal S3. The signals 71 sensed by the magnetic field sensors 39 (e.g., S1 by SAS1 39-1, S2 by SAS2, and S3 by SAS3 39-3) are received by PIU 30 as shown in Fig. 2. The magnetic signals 71 (S1-S3) are processed by PIU 30 and analyzed by processor unit 56 to determine the force applied to tissue 47 (Fig. 1) by distal end expandable assembly 28.

Reference is now made to Fig. 3, which is a schematic view illustrating sensing magnetic signals 73 from a location pad magnetic coil 32 for use in the system of Fig. 1. A less accurate method to estimate the force applied to tissue 47 by distal end expandable assembly 28, but less affected by noise during ablation, includes determining the force from the deformation (i.e., shape of) the distal end expandable assembly 28 based on computing the positions of magnetic field sensors 39 and electro-magnetic coil 29 from magnetic fields generated by magnetic coils 32 of location pad 25 transmitting magnetic signals 73 for detection by magnetic field sensors 39 and electro-magnetic coil 29. The signals 73 sensed by the magnetic field sensors 39 and by electro-magnetic coil 29 are received by PIU 30 as shown in Fig. 3. SAS1 39-1 receives signal S4, SAS2 39-2 receives signal S5, and SAS3 39-3 receives signal S6, and TAS receives signal S7 from the magnetic coils 32. The magnetic signals 73 (S4-S7) are processed by PIU 30 and analyzed by processor unit 56 to compute the positions of the magnetic field sensors 39 and electro-magnetic coil 29, and determine the deformation (i.e., shape) of the distal end expandable assembly 28 from the computed positions, and from the determined deformation or shape the processor unit 56 derives the force applied to tissue 47 by distal end expandable assembly 28.

Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The amount of expandable distal end assembly contraction D_{L} may be computed from the positions of the sensors 29, 30, and based on a known (e.g., measured) spring coefficient K_{L} of expandable distal end assembly 28, a contact force F_{C} can be estimated from **F_{C}=K_{L}·D_{L}.**

The expandable distal end assembly 28 may be tilted or bent from its zero-angle orientation at an angle β. Based on a known (e.g., measured) spring coefficient K_{A} of expandable distal end assembly 28, measured or pre-known distance L between the sensors 29, 30, a contact force F_{A} can be estimated from **F_{A}=K_{A}·L·β.**

When a simple spring model for expandable distal end assembly contraction and tilt is limited (e.g., for some types of expandable distal end assemblies), the processor may use a more complex elastic model (e.g., based on a set of springs) for expandable distal end assembly deformation.

Additionally, or alternatively, the processor can estimate the contact force using empirical data comprising calibration of the forces against expandable distal end assembly tilt and/or deformation. The processor may use weights to interpolate between calibration values. In such a case, a memory of the system is configured to store a relationship between EMC output and contact force based on the empirical data. The processor is configured to use the stored empirical data to relate EMC output to contact force.

### COMBINING FORCE VALUES FROM DIFFERENT METHODS FOR COMPUTING FORCE OF CATHETER EXPANDABLE ASSEMBLY

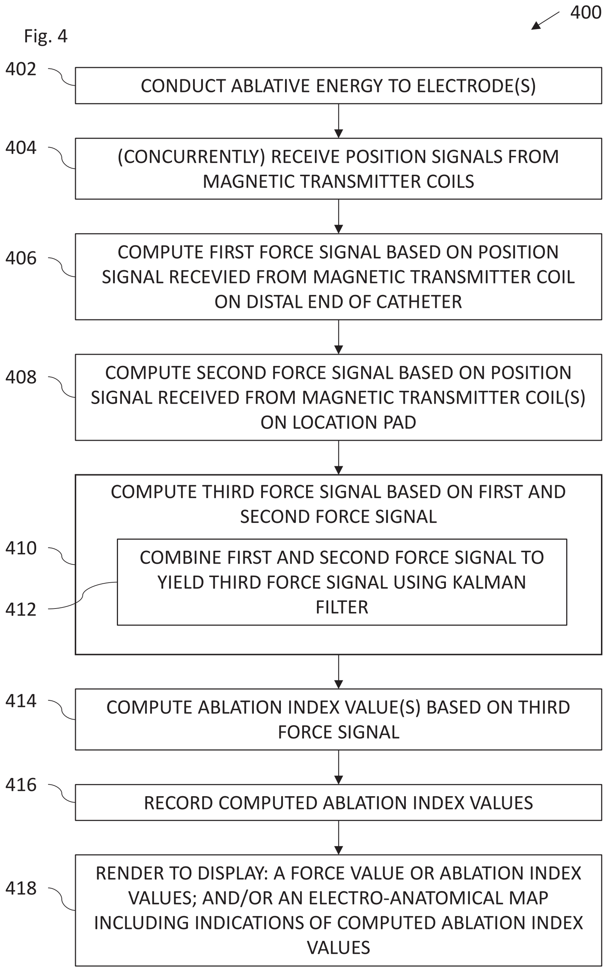

Reference is now made to Fig. 4, which is a flowchart 400 including steps in a method of operation of the system 10 of Fig. 1. The ablation energy generator 50 is configured to conduct ablative energy to a given one of electrodes 26 of catheter 14 (block 402). The processor unit 56 is configured to detect when the ablation energy generator 50 is delivering ablative energy.

Magnetic field sensors 39 are configured to receive the first magnetic signal(s) 71 from magnetic transmitter coil(s) 69; and second magnetic signals 73 from the magnetic transmitter coils 32 disposed in the location pad 25 (block 404). The magnetic field sensor 29 is configured to receive the second magnetic signals 73 from the magnetic transmitter coils 32 disposed in the location pad 25. In some examples, the first magnetic signal(s) 71 and the second magnetic signals 73 are concurrently received by the same magnetic field sensors 39 during the ablation of the tissue by a given one of the electrodes 26. The term concurrently, may include have a gap of up to 300 milliseconds between receipt of pulses of the first and second magnetic signals such as in the range of 10 to 100 milliseconds, or 50 to 150 milliseconds. In some examples, different magnetic-based position sensors 39 may be configured to receive the first and second magnetic signals 71, 73.

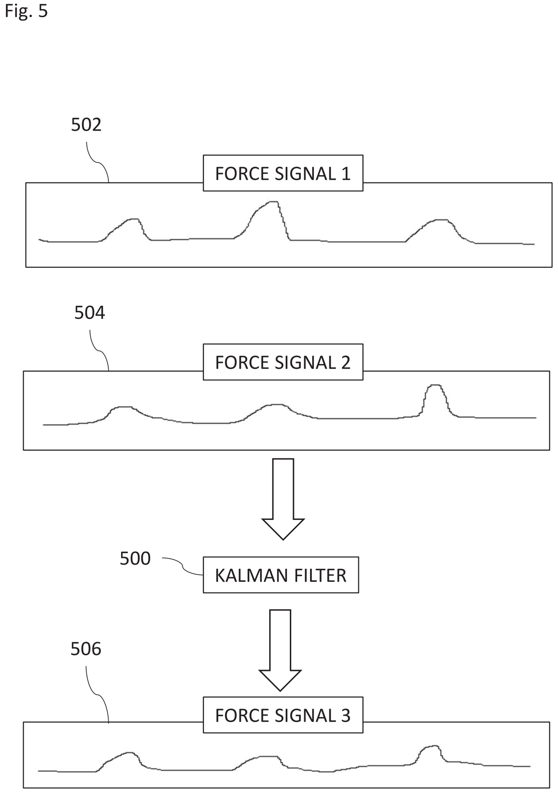

The received magnetic signals 71 may be processed by the PIU 30 and provided to the processor unit 56 to compute a first force signal 502 (Fig. 5) as described in more detail below.

The received magnetic signals 73 may be processed by the PIU 30 and provided to the processor unit 56 to compute positions of the magnetic field sensors 29, 39 receiving the signals 73, and respective shapes (e.g., distorted shapes or distortion) of the distal end assembly 28 over time from the computed positions of the magnetic field sensors 29, 39. The computed distortion (i.e., shapes) of the distal end assembly 28 may be used to compute a second force signal 504 (Fig. 5) as described in more detail below.

The processor unit 56 is configured to compute the first force signal 502 acting on tissue 47 by the distal end assembly of the catheter 14 based on the first magnetic signal(s) 71 received by the first magnetic field sensor(s) 39 of the distal end assembly 28 of the catheter 14 from the magnetic transmitter coil 69 of the distal end assembly 28 of the catheter 14 during ablation of the tissue 47 by the given electrode 26 (block 406).

The processor unit 56 is configured to compute the second force signal 504 acting on tissue 47 by the distal end assembly of the catheter 14 based on the second magnetic signal(s) 73 received by the second magnetic field sensor(s) 29, 39 of the distal end assembly 28 from the magnetic transmitter coils 32 disposed in location pad 25 (block 408). The location pad 25 is configured to be disposed ex-vivo during the ablation of the tissue 47 by the given electrode 26.

Reference is now made to Fig. 5, which is a schematic view of a Kalman filter 500 combining first force signal 502 and second force signal 504 into a third force signal 506. Reference is also made to Fig. 1. The processor unit 56 is configured to compute third force signal 506 based on the first force signal 502 and the second force signal 504 (block 410). In some examples, the processor unit 56 is configured to combine the first force signal 502 and the second force signal 504 to yield the third force signal using Kalman filter 500 (block 412). In some examples, the processor unit 56 is configured to combine the first force signal 502 and the second force signal 504 using the Kalman filter 500 to yield the third force signal 506 as an optimal state estimate (which is mathematically optimal as defined by the Kalman Filter) of force values as a function of time.

Reference is now made to Fig. 8, which is a schematic view of display device 27 showing an electro-anatomical map 600 and various related values. Reference is also made to Fig. 1. In some examples, the processor unit 56 is configured to compute ablation index values 602 based on the third force signal 506, which estimates forces sensed in real-time during ablation at respective ablation sites 604 (block 414). The computed ablation index values provides an indication of quality of the ablation at the respective ablation sites 604. In some examples, processor unit 56 is configured to compute the ablation index values 602 as a function of: the third force signal 506 computed for the respective ablation sites 604; and ablation power used at the respective ablation sites 604 and optionally the number of pulses, the duration of the pulses etc. In some examples, the processor unit 56 is configured to compute the ablation index values based on the third force signal during ablation and other ablation index values based on the first force signal not during ablation.

In some examples, the processor unit 56 is configured to record the computed ablation index values 602 associated with the respective ablation sites 604 (block 416). In some examples, the processor unit 56 is configured render to display device 27 one or more force values 606 (only one shown) or ablation index value(s) 602 computed from the third force signal 506. In some examples, the processor unit 56 is configured to render to display device 27 the electro-anatomical map 600 including an indication of the computed ablation index values 602 associated with the respective ablation sites 604 (block 418).

In some examples, the processor unit 56 is configured to render to the display: during a time period that the ablative energy is being conducted to the catheter 14, the force value 606 or the ablation index value 602 computed from the third force signal 506; and during a time period that the ablative energy is not being conducted to the catheter 14, another force value 606 or another ablation index value 602 computed from the first force signal 502.

In practice, some or all of the functions of processor unit 56 may be combined in a single physical component or, alternatively, implemented using multiple physical components. These physical components may comprise hardwired or programmable devices, or a combination of the two. In some examples, at least some of the functions of the processor unit 56 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

### EXAMPLES

Example 1: An apparatus, comprising an ablation energy generator configured to conduct ablative energy to an electrode of a catheter, a processor configured to compute a first force signal acting on tissue by a distal end assembly of the catheter based on a first magnetic signal received by a first magnetic field sensor of the distal end assembly of the catheter from a magnetic transmitter coil of a distal end of the catheter during ablation of the tissue by the electrode, compute a second force signal acting on tissue by the distal end assembly of the catheter based on a second magnetic signal received by a second magnetic field sensor of the distal end assembly from a magnetic transmitter coil disposed in a location pad configured to be disposed ex-vivo during the ablation of the tissue by the electrode, compute a third force signal based on the first force signal and the second force signal, and render to a display a force value or an ablation index value computed from the third force signal, and a memory configured to store data used by the processor.

Example 2: The apparatus according to example 1, wherein the processor is configured to render to the display during a time period that the ablative energy is being conducted to the catheter, the force value or the ablation index value computed from the third force signal, and during a time period that the ablative energy is not being conducted to the catheter, another force value or another ablation index value computed from the first force signal.

Example 3: The apparatus according to example 1 or 2, wherein the first magnetic field sensor and the second magnetic field sensor are a same magnetic field sensor, wherein the first magnetic signal and the second magnetic signal are concurrently received by the same magnetic field sensor during the ablation of the tissue by the electrode.

Example 4: The apparatus according to any of examples 1-3, wherein the processor is configured to compute the ablation index value based on the third force signal.

Example 5: The apparatus according to any of examples 1-4, wherein the processor is configured to compute the ablation index value based on the third force signal during ablation and an additional ablation index value based on the first force signal not during ablation.

Example 6: The apparatus according to any of examples 1-5, wherein the processor is configured to combine the first force signal and the second force signal to yield the third force signal using a Kalman filter.

Example 7: The apparatus according to example 6, wherein the processor is configured to combine the first force signal and the second force signal using the Kalman filter to yield the third force signal as an optimal state estimate of force values.

Example 8: The apparatus according to any of examples 1-6, further comprising the catheter and the location pad.

Example 9: The apparatus according to any of examples 1-6, wherein the processor is configured to compute ablation index values based on the third force signal estimating forces sensed in real-time during ablation at respective ablation sites, the computed ablation index values providing an indication of quality of the ablation at the respective ablation sites.

Example 10: The apparatus according to example 9, wherein the processor is configured to compute the ablation index values as a function of the third force signal computed for the respective ablation sites, and ablation power used at the respective ablation sites.

Example 11: The apparatus according to example 9, wherein the processor is configured to record the computed ablation index values, and render to a display an electro-anatomical map including an indication of the computed ablation index values associated with the respective ablation sites.

Example 12: A method, comprising conducting ablative energy to an electrode of a catheter, computing a first force signal acting on tissue by a distal end assembly of the catheter based on a first magnetic signal received by a first magnetic field sensor of a distal end assembly of the catheter from a magnetic transmitter coil of a distal end of the catheter during ablation of the tissue by the electrode, computing a second force signal acting on tissue by the distal end assembly of the catheter based on a second magnetic signal received by a second magnetic field sensor of the distal end assembly from a magnetic transmitter coil disposed in a location pad configured to be disposed ex-vivo during the ablation of the tissue by the electrode, computing a third force signal based on the first force signal and the second force signal, and rendering to a display a force value computed from the third force signal or an ablation index value computed from the third force signal.

Example 13: The method according to example 12, further comprising rendering to the display during a time period that the ablative energy is being conducted to the catheter, the force value or the ablation index value computed from the third force signal, and during a time period that the ablative energy is not being conducted to the catheter, another force value or another ablation index value computed from the first force signal.

Example 14: The method according to example 12 or 13, wherein the first magnetic field sensor and the second magnetic field sensor are a same magnetic field sensor, the method further comprising concurrently receiving the first magnetic signal and the second position by the same magnetic field sensor during the ablation of the tissue by the electrode.

Example 15: The method according to any of examples 12-14, further comprising computing the ablation index value based on the third force signal.

Example 16: The method according to any of examples 12-15, further comprising computing the ablation index value based on the third force signal during ablation and an additional ablation index value based on the first force signal not during ablation.

Example 17: The method according to any of examples 12-16, further comprising combining the first force signal and the second force signal to yield the third force signal using a Kalman filter.

Example 18: The method according to example 17, wherein the combining includes combining the first force signal and the second force signal using the Kalman filter to yield the third force signal as an optimal state estimate of force values.

Example 19: The method according to any of examples 12-17, further comprising computing ablation index values based on the third force signal estimating forces sensed in real-time during ablation at respective ablation sites, the computed ablation index values providing an indication of quality of the ablation at the respective ablation sites.

Example 20: The method according to example 19, wherein the computing the ablation index values includes computing the ablation index values as a function of the third force signal computed for the respective ablation sites, and ablation power used at the respective ablation sites.

Example 21: The method according to example 19, further comprising recording the computed ablation index values, and rendering to a display an electro-anatomical map including an indication of the computed ablation index values associated with the respective ablation sites.

Various features of the disclosure which are, for clarity, described in the contexts of separate examples may also be provided in combination in a single example. Conversely, various features of the disclosure which are, for brevity, described in the context of a single example may also be provided separately or in any suitable sub-combination.

The examples described above are cited by way of example, and the present disclosure is not limited by what has been particularly shown and described hereinabove. Rather the scope of the disclosure includes both combinations and subcombinations of the various features described hereinabove, as well as variations and modifications thereof which would occur to persons skilled in the art upon reading the foregoing description and which are not disclosed in the prior art.

## Claims

1. An apparatus, comprising:
an ablation energy generator configured to conduct ablative energy to an electrode of a catheter;
a processor configured to:
compute a first force signal acting on tissue by a distal end assembly of the catheter based on a first magnetic signal received by a first magnetic field sensor of the distal end assembly of the catheter from a magnetic transmitter coil of a distal end of the catheter during ablation of the tissue by the electrode;
compute a second force signal acting on tissue by the distal end assembly of the catheter based on a second magnetic signal received by a second magnetic field sensor of the distal end assembly from a magnetic transmitter coil disposed in a location pad configured to be disposed ex-vivo during the ablation of the tissue by the electrode;
compute a third force signal based on the first force signal and the second force signal; and
render to a display a force value or an ablation index value computed from the third force signal; and
a memory configured to store data used by the processor.

2. The apparatus according to claim 1, wherein the processor is configured to render to the display:
during a time period that the ablative energy is being conducted to the catheter, the force value or the ablation index value computed from the third force signal; and
during a time period that the ablative energy is not being conducted to the catheter, another force value or another ablation index value computed from the first force signal.

3. The apparatus according to claim 1, wherein the first magnetic field sensor and the second magnetic field sensor are a same magnetic field sensor, wherein the first magnetic signal and the second magnetic signal are concurrently received by the same magnetic field sensor during the ablation of the tissue by the electrode.

4. The apparatus according to claim 1, wherein the processor is configured to compute the ablation index value based on the third force signal.

5. The apparatus according to claim 1, wherein the processor is configured to compute the ablation index value based on the third force signal during ablation and an additional ablation index value based on the first force signal not during ablation.

6. The apparatus according to claim 1, wherein the processor is configured to combine the first force signal and the second force signal to yield the third force signal using a Kalman filter.

7. The apparatus according to claim 6, wherein the processor is configured to combine the first force signal and the second force signal using the Kalman filter to yield the third force signal as an optimal state estimate of force values.

8. The apparatus according to claim 1, further comprising the catheter and the location pad.

9. The apparatus according to claim 1, wherein the processor is configured to compute ablation index values based on the third force signal estimating forces sensed in real-time during ablation at respective ablation sites, the computed ablation index values providing an indication of quality of the ablation at the respective ablation sites.

10. The apparatus according to claim 9, wherein the processor is configured to compute the ablation index values as a function of: the third force signal computed for the respective ablation sites; and ablation power used at the respective ablation sites.

11. The apparatus according to claim 9, wherein the processor is configured to:
record the computed ablation index values; and
render to a display an electro-anatomical map including an indication of the computed ablation index values associated with the respective ablation sites.
